(19) Europäisches Patentamt / European Patent Office / Office européen des brevets

(11) **EP 2 133 453 B1**

(12) **EUROPEAN PATENT SPECIFICATION**

(45) Date of publication and mention
of the grant of the patent:
**22.06.2016 Bulletin 2016/25**

(21) Application number: **08722678.3**

(22) Date of filing: **24.03.2008**

(51) Int Cl.:
*B32B 5/02* (2006.01)     *B32B 5/04* (2006.01)
*B32B 5/08* (2006.01)     *B32B 7/12* (2006.01)
*B32B 5/26* (2006.01)     *B32B 5/14* (2006.01)
*D04H 1/42* (2012.01)     *D04H 1/70* (2012.01)
*A61F 13/49* (2006.01)     *D04H 13/00* (2006.01)

(86) International application number:
**PCT/JP2008/055362**

(87) International publication number:
**WO 2008/123169 (16.10.2008 Gazette 2008/42)**

(54) **NONWOVEN STRETCH FABRIC**

ELASTISCHER VLIESSTOFF

TISSU EXTENSIBLE NON TISSÉ

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **30.03.2007 JP 2007094903**

(43) Date of publication of application:
**16.12.2009 Bulletin 2009/51**

(73) Proprietor: **Unicharm Corporation
Ehime 799-0111 (JP)**

(72) Inventors:
• **MITSUNO, Satoshi
Kanonji-shi
Kagawa 769-1602 (JP)**

• **GODA, Hiroki
Kanonji-shi
Kagawa 769-1602 (JP)**

(74) Representative: **Sperling, Rüdiger et al
Staeger & Sperling
Partnerschaftsgesellschaft mbB
Sonnenstrasse 19
80331 München (DE)**

(56) References cited:
**WO-A1-2006/115251**    **GB-A- 1 510 580**
**JP-A- 2003 073 967**    **JP-A- 2004 131 918**
**JP-A- 2004 244 791**    **JP-A- 2005 089 870**
**JP-A- 2006 045 291**

**Description**

TECHNICAL FIELD

The present invention relates to an elastic nonwoven fabric.

BACKGROUND ART

Conventionally, nonwoven fabrics have been used in wide variety of fields including absorbent articles such as disposable diapers and sanitary napkins, cleaning products such as wipers, and medical supplies such as masks. Although nonwoven fabrics are used in various fields as described above, in cases where these fabrics are being employed as products in each of the abovementioned fields, it must be manufactured so as to have characteristics and structures suitable for each product application therefor.

For example, it is important that when employed in absorbant articles, the nonwoven fabric employed therein feels comfortable when worn and stretches in accordance with the body movement(s) of a wear. Furthermore, when nonwoven fabrics are used for disposable diapers, for example, these fabrics must are required to have a soft texture and superior breathability, and sufficient strength to prevent sheets from breaking during elongation while maintaining the elasticity thereof.

Accordingly, nonwoven fabrics are disclosed in which the texture and elasticity thereof is improved via an elongating treatment to the partially fused mixed fibers (e.g., refer to Patent Document 1). The nonwoven fabrics disclosed in Patent Document 1 are partially fused mixed fibers elongated by using 2 or more nip rolls, and accelerating the rotational speed in sequence in the machine direction of the device.

Patent Document 1: Japanese Patent Application Laid-Open (JP-A) No. 2004-244791

Further, in the field of the invention prior art WO2006/115251 A1 discloses a nonwoven stretch fabric and method for producing the same.

DISCLOSURE OF THE INVENTION

Problems to be Solved by the Invention

However, the pre-elongation processed nonwoven fabrics (nonwoven fabrics with a latent elasticity) disclosed In Patent Document 1 have a fiber diameter as large as 24 $\mu$m to 26 $\mu$m, such that when the nonwoven fabrics with a low basis weight of 50 g/m$^2$ or less that are not disclosed in Patent Document 1 are being elongated, the portions with partially low basis weight are expressed. Accordingly, the expression of the portions with partially low basis weight described above would cause the nonwoven fabrics to break when elongated by an even higher elongation ratio, and therefore, the predefined degree of elongation necessary to be employed in wearable products could not have been achieved.

The present invention has been implemented to solve the above problems, and therefore, an object of the present invention is to provide nonwoven fabrics with a comfortable texture (cushioning property) and superior breathability, suppress breakage or weakening in the strength thereof, which can occur during the elongation treatment, and to provide a wide range of elasticity (stretchability) during use, even when the nonwoven fabric has a low basis weight.

Means for Solving the Problems

The present inventors have discovered that nonwoven fabrics having a comfortable texture (cushioning property) and superior breathability can be obtained by the formation of elastic nonwoven fabrics via mixing or layering of elongatable fibers with stretchable fibers, so that the elongatable fibers having a smaller fiber diameter are arranged in predefined regions, that these fibers provide a superior range of elasticity during wear, even in nonwoven fabrics with a low basis weight; and that breakage and weakening in the strength thereof, which occur during the elongation treatment, can be suppressed, to thereby complete the present invention. Specifically, the below-mentioned nonwoven fabrics are provided.

In a first aspect of the present invention, an elastic nonwoven fabric having a longitudinal direction and a width direction perpendicular to the longitudinal direction thereof, includes: a plurality of belt-like low-density regions extending in the longitudinal direction; a plurality of belt-like high-density regions extending in the longitudinal direction, in which the belt-like low-density regions and the belt-like high-density regions are formed on both faces of the elastic nonwoven fabric, alternately and continuously formed in the width direction, and in which the belt-like high-density regions on one face and the belt-like high density regions on the other face are formed alternately in the width direction; thermoplastic polyolefin elongatable fibers; and thermoplastic elastomer stretchable fibers mixed or layered with the thermoplastic polyolefin elongatable fibers. The thermoplastic polyolefin elongatable fibers include partially elongated fibers between the belt-like high-density regions on one face and the belt-like high-density regions on the other face, and the average fiber diameter of the fibers configuring the partially elongated fibers is 12 $\mu$m to 21 $\mu$m, and wherein a nonwoven fabric sheet, which is a nonwoven fabric with a latent elasticity, is subjected to elongation processing in order to form the elastic nonwoven fabric, the nonwoven fabric having latent elasticity contains thermoplastic polyolefin elongatable fibers and thermoplastic elastomer stretchable fibers mixed and fixed to one another by self-fusion induced by being softened or fused at plurality of fused portions arranged at regular intervals.

In a second aspect of the present invention, the elastic nonwoven fabric has a basis weight of less than 50 g/m$^2$.

In a third aspect of the present invention, a composite sheet includes: the elastic nonwoven fabric according to claim 1 or 2; a non-elastic nonwoven fabric; a joint portion whereby the elastic nonwoven fabric and the non-elastic nonwoven fabric are joined intermittently along a direction of streching; and a non-joint portions between the joint portion and an adjacent joint portion in which a plurality of wrinkles are formed on a side not in contact with a skin along a direction intersecting the direction of streching by relaxiation of a non-elastic sheet when the elastic nonwoven fabric is not being elongated.

In a fourth aspect of the present invention, an absorbent article includes: a chassis with front and rear portions, and an absorbent body with liquid retaining capacity, in which at least a part of the chassis comprises the composite sheet according to claim 3, and the composite sheet thereof is arranged on a side in contact with the skin at a time the absorbent article is worn.

Effects of the Invention

The present invention provides a nonwoven fabric with superior texture (cushioning property) and breathability obtained by forming an elastic nonwoven fabric via mixing or layering of elongatable fibers and stretchable fibers, such that the elongatable fibers having a smaller fiber diameter are arranged in predefined regions, to thereby allow for nonwoven fabrics with a low basis weight to have a wide range of elasticity during wear and to suppress breakage and weakening of strength caused by the elongation thereof.

BRIEF DESCRIPTION OF THE DRAWINGS

Fig. 1 shows a perspective view of elastic nonwoven fabric 1 according to the present invention;
Fig. 2 shows a cross-sectional view of a frbrous state of the elastic nonwoven fabric 1;
Fig. 3 shows a relationship between the range of elasticity of elastic nonwoven fabric 1 after elongation and the elongation ratio applied to nonwoven fabric sheet 2 before elongation;
Fig. 4 shows a relationship between the degree of elasticity and the strength in the width direction of the elastic nonwoven fabric 1;
Fig. 5 shows a relationship between the degree of crystallinity and the distortion factor of the polyolefin fibers after 100% cycle of single fiber;
Fig. 6 is a view describing a method of manufacturing the elastic nonwoven fabric 1;
Fig. 7A is a view illustrating the nonwoven fabric sheet 2 while being subjected to a gear elongation process;
Fig. 7B is a partially enlarged view of an engagement portion in Fig. 7A;
Fig. 8 shows a relationship between the fiber diameter, the basis weight and compression work rate of the elastic nonwoven fabric 1;
Fig. 9 shows a relationship between the fiber diameter, the basis weight and degree of air-flow resistance of the elastic nonwoven fabric 1;
Fig. 10 shows a relationship between the degree of strength in the width direction of the elastic nonwoven fabric sheet 2 and the processing speed;
Fig. 11 shows a relationship between the degree of strength in the width direction of the nonwoven fabric sheet 2 and the temperature of the nonwoven fabric sheet 2;
Fig. 12 is a frontal view showing a pull-up type disposable diaper 50 as an example of the absorbent article using a composite sheet 4 produced by sticking the elastic nonwoven fabric 1 of the present invention and a non-elastic nonwoven fabric together; and
Fig. 13 is a development view of the disposable diaper 50.

PREFERRED MODE FOR CARRYING OUT THE INVENTION

Embodiments of the present invention will be described hereinafter. In the present invention, a nonwoven fabric sheet, which is a nonwoven fabric with a latent elasticity, is subjected to elongation processing in order to form an elastic nonwoven fabric. The nonwoven fabric having latent elasticity contains elongatable fibers and stretchable fibers mixed and fixed to one another by self-fusion induced by being softened or fused at plurality of fused portions arranged at regular intervals. Moreover, the elongatable fibers described herein are, for example, fibers which undergo plastic deformation by having a lower elasticity than the elasticty at a maximum strength of the stretchable fibers. For example, the elongatable fibers are fibers which are expandable with small elasticity, and the stretchable fibers include fibers which can be elastically elongated.

Moreover, in present embodiment, since the nonwoven fabric sheet 2 is heated by a gear elongation process described below, the thermoplastic polyolefin fibers is described as elongatable fibers.

Stretchable Nonwoven Fabric

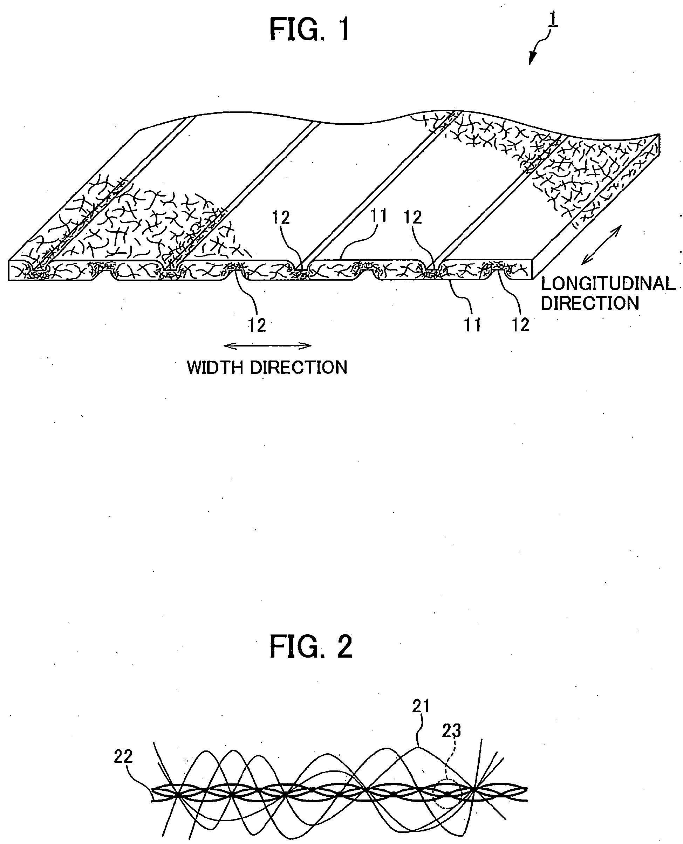

The elastic nonwoven fabric 1 of the present embodiment will be explained with reference to Figs. 1 and 2.

As shown in Fig. 1, the elastic nonwoven fabric 1 has a longitudinal direction and a width direction, and is formed into a sheet-like form. Furthermore, the elastic nonwoven fabric 1 is formed by mixing the thermoplastic polyolefin fibers 21 which can be elongated and the stretchable thermoplastic elastomer fibers 22, and alternatively forming low-density regions 11 with a plurality of belt-like low-density regions extending in the longitudinal direction, and high-density regions 12 with a plurality of belt-like high-density regions extending in the longitudinal direction, on both faces of the elastic nonwoven fabric. Accordingly, the high-density regions 12 on one face and the high-density regions 12 on the other face are formed in such a way that the high-density regions 12 formed on one face and the high-density regions 12 formed on the other face alternate respectively in the width direction of the elastic nonwoven fabric 1. Similarly, the low-density regions 11 on one face and the low-density regions 11 on the other face are formed in such a way that the low-density regions 11 formed on one face and the low-density regions 11 formed on the other face alternate respectively in the width direction of the elastic nonwoven fabric 1.

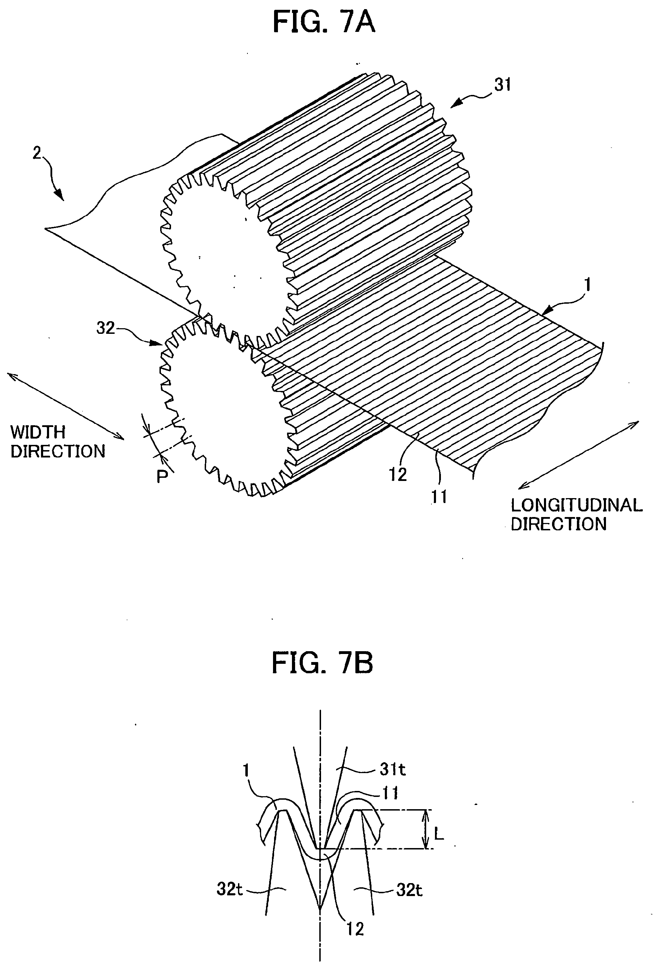

The elastic nonwoven fabric 1 is formed by machining the nonwoven fabric sheet 2 through the gap between a pair of gear rolls 31 and 32 in the gear elongation process described below (Fig. 7A), and is provided with elasticity (stretchability) via the gear elongation processing. Specifically, the nonwoven fabric sheet 2 machined through the gap between the gear rolls 31 and 32 is compressed in three point bending by the gear teeth 31t and 32t formed by the gear rolls 31 and 32 (refer to Fig. 7B). Accordingly, the polyolefin fibers 21 and the elastomer fibers 22 making up the elastic nonwoven fabric 1 are elongated. The high-density regions 12 are then formed in the elastic nonwoven fabric 1, which is formed by performing gear elongation processing on the nonwoven fabric sheet 2, by compressing the elastic nonwoven fabric 1 with the top portion of the interlocking gears teeth 31t and 32t in meshing engagement. Here, since the high-density regions 12 are compressed by gear teeth 31t and 32t, the non-elongated polyolefin fibers 21 are arranged in the high-density regions 12.

In addition, the low-density regions 11 are formed in the portions of the elastic nonwoven fabric 1 not compressed by gear teeth 31t and 32t (portions abutting the sides of gear teeth 31t and 32t) because the polyolefin fibers 21 are elongated by the interlocking gear teeth 31t and 32t in meshing engagement with one another. Accordingly, the polyolefin fibers 21 elongated by the gear elongation described below are arranged in the low-density regions 11.

Consequently, the high-density regions 12 contain more non-elongated polyolefin fibers 21 than the low-density regions 11, and the low-density regions 11 contain more polyolefin fibers 21 elongated by the gear elongation process described below than the high-density regions 12.

Although the elastomer fibers 22 in the low-density regions 11 contract back to the original fiber length thereof even after gear elongation, the polyolefin fibers 21 elongated by the gear elongating process do not contract back to original fiber length thereof, and are at least partially elongated by gear elongating processing. The polyolefin fibers 21 are then expanded in the thickness direction with the elongated portion thereof being centered at fused portion 23 (refer to Fig. 2). Specifically, the thickness of the low-density regions 11, as the thickness of the elastic nonwoven fabric 1, is increased. This is because the polyolefin fibers 21 are elongated and the void in the elastic nonwoven fabric 1 is increased more than that of the elastic nonwoven fabric 1 before gear elongation processing.

Once the elastic nonwoven fabric formed abovementioned manner is released from elongation device, elongatable areas are formed to contain the elasticity of the elastomer fibers 22 and the elongated polyolefin fibers 21, thus making the elongation possible. In addition, the thickness is increased in the low-density regions 11 due to the voids, thereby improving the texture (cushioning property).

Moreover, the fiber diameter of the elongated portions of the elongated polyolefin fibers 21 becomes smaller. The fiber diameter of the elongated polyolefin fibers are preferably formed with a fiber diameter in the range of 12 μm and 21 μm. In cases where the fiber diameter is less than 12 μm, the polyolefin fibers 21 may be cut during use, and when the fiber diameter is more than 21μm, cushioning property or breathability may be degraded due to the increased density of the elastic nonwoven fabric.

The elastic nonwoven fabric 1 is formed of the above-mentioned fiber structure, and may be used for the nonwoven fabric with a basis weight of 50 g/m$^2$ or less in a relaxed state. Specifically, it may be used for the nonwoven fabric with a basis weight of 50 g/m$^2$ to 15 g/m$^2$. In cases where the basis weight of the elastic nonwoven fabric 1 is less than 15 g/m$^2$, the elastic nonwoven fabric 1 may break easily after forming, and when the elastic nonwoven fabric 1 is bonded with the non-elastic sheet to use as a composite sheet, the adhesive agent in use may leak out when sticking the two together.

Moreover, the basis weight of the polyolefin fibers 21 in the elastic nonwoven fabric 1 in the relaxed state may be in the range of 4 g/m$^2$ to 40 g/m$^2$, for example. In cases where the basis weight is less than 4 g/m$^2$, obtaining a superior texture is difficult, and when the basis weight is more than 40 g/m$^2$, the fabric may be too rigid for the elastic nonwoven fabric 1. The basis weight of the elastomer fibers 22 in the elastic nonwoven fabric 1 may be in the range of 3 g/m$^2$ to 38 g/m$^2$, for example. In cases where the basis weight is less than 3 g/m$^2$, achieving sufficient elasticity for the elastic nonwoven fabric 1 is difficult, and when the basis weight is more than 38 g/m$^2$, the fibers are not practical as an elastic nonwoven fabric 1 due to too much elastic force at the time of wear.

Furthermore, it is preferable for the amount of distortion in the 100% cycle test of the elastic nonwoven fabric 1 to be 20% or less when the elastic nonwoven fabric 1 is formed under the abovementioned conditions. In cases where the amount of strain intensity is more than 20%, the shrinkage dimensions of products becomes unstable and variations in the comfort of the wearer may occur when the elastic nonwoven fabric 1 is employed in an absorbent article.

Moreover, examples of the thermoplastic polyolefin fibers 21 of the present embodiment may include single fibers such as polypropylene fibers or polyethylene fibers, or composite fibers composed of a core-in-sheath structure containing polypropylene or polyethylene.

Examples of the elastomer fibers 22 used for the elastic nonwoven fabric 1 may include fibers such as a polyurethane-based elastomer, a polystyrene-based elastomer, a polyolefin-based elastomer, a polyamide-based elastomer and a polyether-based elastomer. However, among these polyurethane-based elastomer is preferably employed.

Furthermore, a mixing ratio (ratio by weight) of the polyolefin fibers 21 and the elastomer fibers 22 may range from 80:20 to 25:75, for example. In cases where the mixing ratio of polyolefin fibers 21 is more than 80%, the distortion of the elastic nonwoven fabric 1 may be increased, and in cases where the mixing ratio of elastomer fibers 22 is more than 75%, a sticky sensation may occur.

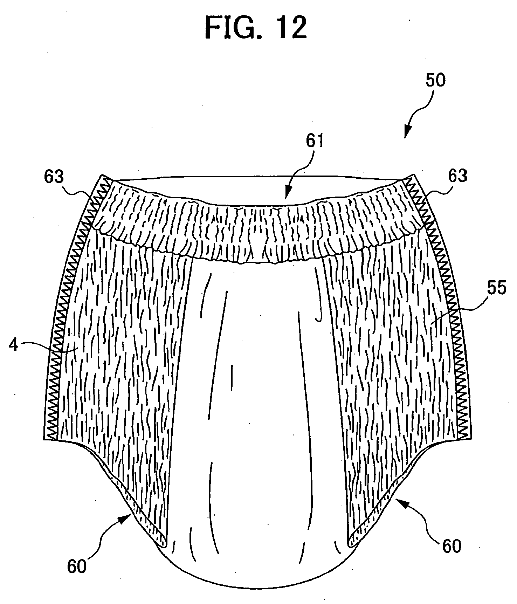

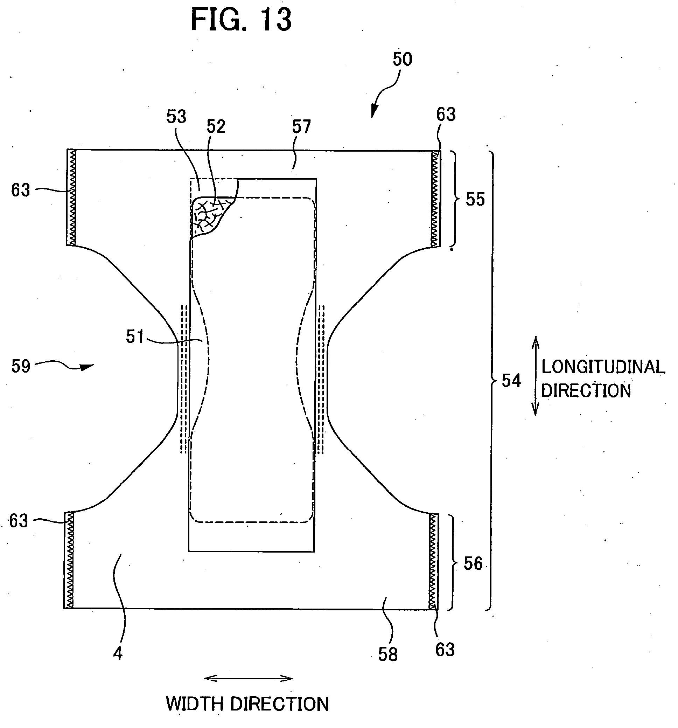

Next, the elastic nonwoven fabric 1 of the present invention will be explained with regard to cases where employed for an absorbent article, the disposable diaper 50. Fig. 12 is a frontal view showing a pull-up type disposable diaper 50 as an exemplary absorbent article using a composite sheet 4 produced by bonding the elastic nonwoven fabric 1 of the present invention with a non-elastic nonwoven fabric; and Fig. 13 is a development view of the disposable diaper 50. The disposable diaper 50 contains a chassis 54 forming the body; a liquid permeable front side sheet 51 disposed on the side of the chassis 54 in contact with the skin; a liquid impermeable back side sheet 53 disposed on the side of the chassis 54 not in contact the skin; and a liquid retainable absorbent body core 52 arranged between the front side sheet 51 and the chassis 54.

The chassis 54 is formed in the form of pull-ups with a front portion 55 and a back portion 56, a front waist gathering 57 which forms the edge of the front portion 55, and a back waist gathering 58 which forms the edge of the back portion 56. A substantially U-shaped cut portion 59 is then formed on the inner side of the chassis 54 with both ends in the width direction of the chassis 54 of the development view, and the approximate center in the longitudinal direction, as a top. The cut portion 59 forms a leg opening 60 when the front portion 55 and the back portion 56 of the chassis 54 are joined together with at a joint portion 63 to form a pull-up shape. Moreover, a waist opening 61 is formed by joining the front portion 55 and the back portion 56 together.

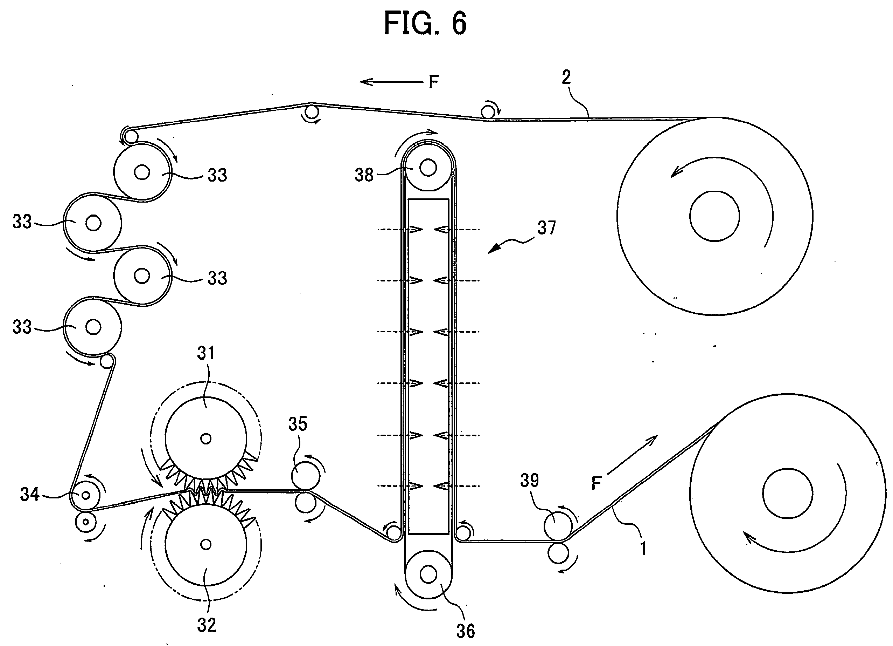

Meanwhile, a composite sheet 4 produced by bonding the non-elastic sheet to the elastic non-woven fabric 1 with an adhesive bond is used as the chassis 54 in the present embodiment. The composite sheet 4 is arranged so that the flow direction F in Fig. 6 corresponds to the width direction in Fig. 13. Specifically, the composite sheet 4 contains a joint portion where the elastic nonwoven fabric 1 and the non-elastic sheet (one example of the non-elastic nonwoven fabric of the present invention) are joined intermittently along the direction of stretching, and a number of wrinkles (folds) on the side not in contact with the skin formed along the direction intersecting the direction of stretching by relaxing of the non-elastic sheet at the non-joint portions between joint portions when the elastic nonwoven fabric 1 is not being stretched (refer to Fig. 12). This allows the disposable diaper 50 to have an elasticity along the periphery of the diaper via the composite sheet 4 when the diaper is produced by joining the front portion 55 and the back portion 56 to form a pull-up shape. Therefore, the disposable diaper 50 exhibits an elasticity according to the bodily movement during wearing without arranging elastic members, such as rubber threads, in the waist gatherings 57 and 58. By eliminating the necessity for elastic members, physical irritation to the skin can be reduced. Naturally, according to the present invention, elastic members also may be disposed in the waist gathering or leg gathering, when necessary.

In this embodiment, the composite sheet 4 is arranged such that the elastic nonwoven fabric 1 is opposing the side in contact with the skin, and wrinkles (folds) formed by the non-elastic sheet appear on the surface, as shown in Fig. 12. Such an arrangement of the composite sheet 4 makes increasing the contact area ratio with the skin possible.

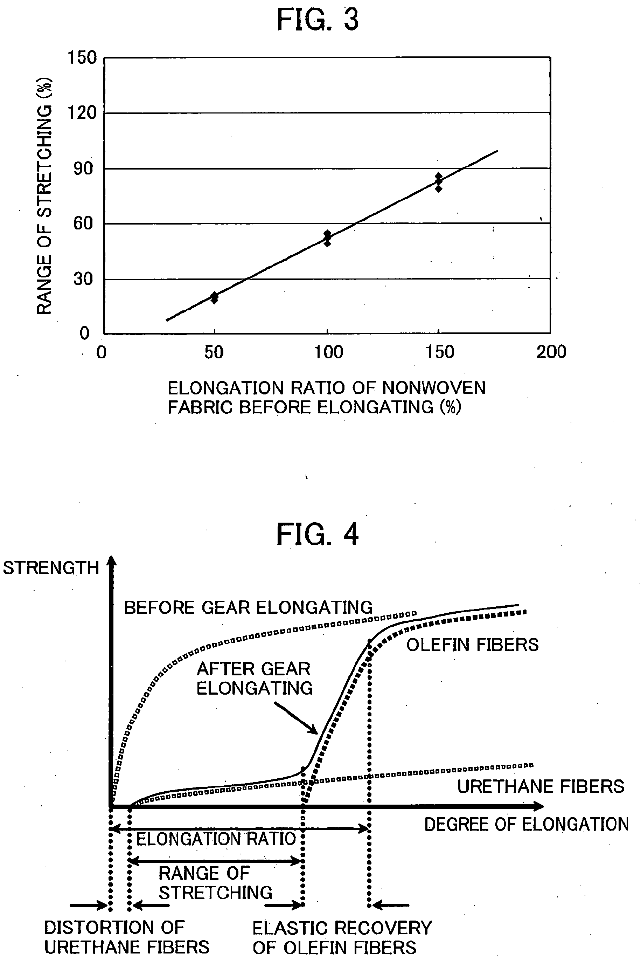

In cases where the elastic nonwoven fabric 1 is used for the disposable diapers, the basis weight is preferably 50 g/m² or less, in order for the elastic nonwoven fabric 1 to be stretched at lower intensity (intensity of 10 N/50 mm at 75% elongation). Moreover, the range of elasticity must be 1.7 times or more (70%), at least for the post-elongation treatment elastic nonwoven fabric 1. The relationship between the range of elasticity (%) of the elastic nonwoven fabric 1 after elongation and the elongation ratio (%) provided to the nonwoven fabric sheet 2 before elongation is shown in Fig. 3.

As shown in Fig. 3, the formation of nonwoven fabric sheet 2 is necessary so to allow greater elongation in order to increase the range of elasticity of the elastic nonwoven fabric 1 after elongation. Specifically, elongation processing is necessary such that the pre-elongation treatment nonwoven fabric sheet 2 can be highly elongated. For example, elongation processing is necessary such that at least the pre-elongation the nonwoven fabric sheet 2 is elongated 2.6 times (160%) in order to provide the post-elongation elastic nonwoven fabric 1 with the range of elasticity of 1.8 times (80%) or more.

However, in cases where the pre-elongation nonwoven fabric sheet 2 is highly elongated, and in cases where the constituent materials of the nonwoven fabric sheet 2 are the elastomer fibers 22 such as urethane fiber and polyolefin fibers 21, for example, the elastomer fibers 22 can be elongated due a high degree of elongation, whereas the polyolefin fibers 21 have a low degree of fiber elongation and a high degree of strength, possibly causing broken fibers or damage at the fused portions, such as fused portions 23, where the polyolefin fibers 21 and the elastomer fibers 22 are fused together. This is likely to cause fluffing from the broken fibers, as well as a loss of strength in the post-elongation elastic nonwoven fabric during elongating, and breakage during processing. Therefore, the olefin fibers and the nonwoven fabric sheet 2 that have a high degree of elasticity are needed to perform elongation at high elongation ratio.

In order to obtain such highly elastic olefin fibers, fibers may be obtained by a method of producing fibers with a relatively low fiber forming rate using resins such as polypropylene or polyethylene, by dry blending polyethylene into polyethylene-polypropylene copolymer or polypropylene with a low degree of crystallinity, or by using an olefinic elastomer to form fibers.

An embossed area ratio in the fused portions 23 of the elastomer fibers 22 and the polyolefin fibers 21 may be reduced, and the sheet may be manufactured by spun lace method or needle punch method, in which fibers are not heat-sealed, but mechanically winded in order to increase the degree of elongation of the nonwoven fabric sheet 2.

Moreover, the elasticity range of the elastic nonwoven fabric 1 may be widened also by increasing distortion of the polyolefin fibers 21 after elongating. The relationship between the degree of elongation (%) and the strength for each type of nonwoven fabric and fiber in the width direction of the elastic nonwoven fabric 1 is shown in Fig. 4. As can be seen from Fig 4, the elasticity range of the elastic nonwoven fabric 1 can also be effectively obtained with a low elongation ratio by decreasing the elastic recovery of the polyolefin fibers 21 as shown by the mathematical formula below.

$$\text{Range of elasticity} = \text{Elongation Ratio} - \text{Distortion of}$$

$$\text{Elastomer Fiber (Urethane Fiber)} - \text{Elastic Recovery of}$$

$$\text{Polyolefin Fiber}$$

$$\text{Elastic Recovery of Polyolefin Fiber} = \text{Elongation Ratio} -$$

$$\text{Distortion of Polyolefin Fiber}$$

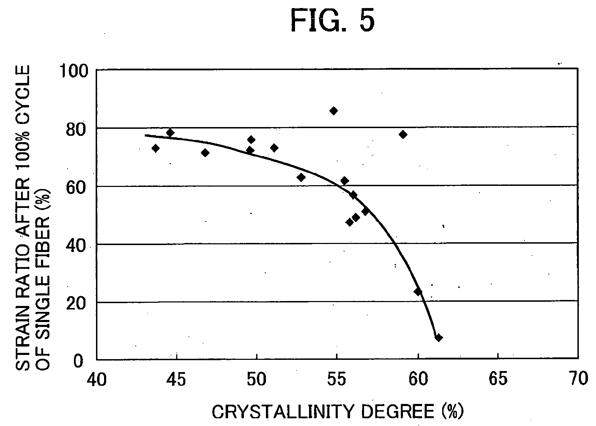

Furthermore, degree of crystallinity of the polyolefin fibers 21 may be decreased in order to reduce the elastic recovery of the polyolefin fibers 21 and increase the distortion thereof. The relationship between the degree of crystallinity of the polyolefin fibers and the distortion ratio after 100% cycle of single fiber of the polyolefin fibers is shown in Fig. 5. When the degree of crystallinity is more than 57%, the distortion of the polyolefin fibers is reduced. Since this makes it hard to obtain the range of elasticity as well as to increase the fiber strength, the degree of sheet elongation is likely to be decreased.

However, in order to decrease the degree of crystallinity of the fibers, the fiber draft ratio during fiber formation may be reduced or fiber formation may be performed at high temperature. Moreover, the resins employed may include resins in which polyolefin copolymer or polypropylene with relatively low orientation is blended with polyethylene, and olefinic elastomers. Since the fibers with a low degree of crystallinity have a low elastic recovery and a high degree of fiber elongation, the nonwoven fabric sheet 2 consisting of such fibers has a high degree of sheet elongation without any weakening of strength during elongating, and therefore are preferable.

Method of Manufacturing

Next, a method of manufacturing the elastic nonwoven fabric 1 will be explained.

Fig. 6 shows an explanatory diagram of the method of manufacturing the elastic nonwoven fabric 1. As shown in Fig. 6, the method of manufacturing the elastic nonwoven fabric 1 includes: an unwinding step, in which the nonwoven fabric sheet 2 is unwound from a nonwoven fabric roll , (a nonwoven fabric sheet as a material rolled into a roll) continuously in the form of sheet along the flow direction F; a heating step in which unwound nonwoven fabric sheet 2 is heated while being transported in the flow direction F; a preliminary elongation step, in which the nonwoven fabric sheet 2 heated to a higher temperature is elongated preliminarily by applying a predetermined tensile force to the nonwoven fabric sheet 2 in the flow direction F, a gear elongation step, in which the nonwoven fabric sheet 2 elongated preliminarily is further elongated in the flow direction F by means of gear rolls, a cooling step, in which the nonwoven fabric sheet 2 (the elastic nonwoven fabric 1) elongated by gear rolls is cooled, and a winding step in which the elastic nonwoven fabric 1, which is the cooled nonwoven fabric sheet 2, is wound into a roll.

First, in the unwinding step, the nonwoven fabric sheet 2 is unwound from the nonwoven fabric roll in which a winding equipment for winding is disposed. The unwound nonwoven fabric sheet 2 is then conveyed at a predefined standard speed, and fed continuously to the heating step located downstream of the flow direction F in the form of a sheet.

In the heating step, a heater is disposed for heating the nonwoven fabric sheet 2 being transported. Specifically, four heating rollers 33 are arranged as the heater. The nonwoven fabric sheet 2 in the form of a sheet is sent sequentially to each heating roller 33 while being winded about the flat peripheral surface of each heating roller 33 forming an approximate S-shape. The nonwoven fabric sheet 2 is then heated by the peripheral surface of the heating rollers 33 while in contact with the flat peripheral surfaces of these heating rollers 33.

A heat generator for heating the peripheral surface is disposed inside each heating roller 33, and the regulation of the temperature of the peripheral surface as well as that of the nonwoven fabric sheet 2 is possible by regulating the amount of heat generated by the heat generator. The temperature of the peripheral surface and that of the nonwoven fabric sheet 2 vary depending on the fiber structure of the nonwoven fabric sheet 2, and in the case of thermoplastic polyolefin fibers, temperatures are regulated so as to be below the melting point of the polyolefin fibers. For example, the temperature of the nonwoven fabric sheet 2 is no more than the melting point of polyolefin fibers, and preferably 40°C or more. In cases where the temperature is 40°C or less, the elasticity for elongation of the fibers is insufficient and there is a reduction in the strength thereof. Moreover, in cases where the temperature is greater than the melting point of olefin fibers, fibers stick to the heating rollers 33 or gear rolls 31 and 32, possibly causing melting and cutting of the nonwoven fabric sheet 2 during elongating.

The nonwoven fabric sheet 2 heated in the heating step is sent to the preliminary elongation step. The preliminary elongation step is performed by setting different peripheral speeds for the heating rollers 33 in the heating step and the gear rolls 31 and 32 in the gear elongating step, respectively. More specifically, the peripheral speed of the gear rolls 31 and 32 are set higher than the peripheral speed of the heating rollers 33, so that the tensile force for preliminary elongating is applied to the nonwoven fabric sheet 2.

In the present embodiment, a guide roller 34 is disposed between the heating rollers 33 and the gear rolls 31 and 32 such that the nonwoven fabric sheet 2 is guided through the roll gap of the gear rolls 31 and 32. The nonwoven fabric sheet 2 is configured to wind about the guide roller 34 just at a predetermined winding angle, such that the flow direction F is oriented toward the roll gap of the gear rolls 31 and 32.

In addition, the nonwoven fabric sheet 2 in the preliminary elongating, for example, is elongated 1.1 to 1.8 times with respect to the flow direction F by the difference in peripheral speeds of the above-mentioned heating rollers 33 and the gear rolls 31 and 32. An increase in the elongation ratio increases the tensile force applied to the nonwoven fabric sheet 2, possibly causing fractures. However, in the embodiment, the nonwoven fabric sheet 2 is preheated in the above-mentioned heating step, and the temperature of the nonwoven fabric sheet 2 is therefore increased. Furthermore, since the nonwoven fabric sheet 2 contains thermoplastic polyolefin fibers, the thermoplastic polyolefin fibers are likely to undergo plastic deformation via the increased temperature. Consequently, breakage of the nonwoven fabric sheet 2 can be prevented during the preliminary elongating step. Meanwhile, the elongation ratio in the preliminary elongating step may be appropriately changed according to other various conditions.

In the gear elongating step, in addition to the amount of elongation applied in the preliminary elongating step, the nonwoven fabric sheet 2 is further elongated in the flow direction F by means of the gear rolls 31 and 32. Hereinafter the process of gear elongation will be explained. As shown in Fig. 7A, the gear elongating is performed by using a pair of gear rolls 31 and 32 with gear teeth 31t and 32t on the peripheral surfaces formed to be corrugated in the peripheral direction at a predefined pitch P. More specifically, in the gear elongating, the nonwoven fabric sheet 2 is deformed in three-point bending by means of the gear tooth 31t of interlocking upper gear roll 31 and the gear tooth 32t of lower gear roll 32 which are in meshing engagement with one another as the nonwoven fabric sheet 2 is machined through the gap between these gear rolls 31 and 32, thereby elongating the nonwoven fabric sheet 2 in the flow direction F.

In cases where the nonwoven fabric sheet 2 is machined through the roll gap, the length of a part of the nonwoven fabric sheet 2 before gear elongating, which is the pitch 2 as mentioned above, is deformed in three-point bending and elongated by means of the gear teeth 31t and 32t which are in meshing engagement with one another. This change in the state can be expressed by the mathematical formula below, in which Mg represents a function of the elongation ratio of the nonwoven fabric sheet 2 by gear elongation, P represents the pitch of the gear teeth 31t and 32t, and L represents the engagement length between the gear teeth 31t and 32t. Partially elongated fibers with elongation ability are formed between the belt-like high density region on one face and the belt-like high-density region on the other face of the present invention via the abovementioned elongating.

$$Mg = 2 \times ((L^2 + (P/2)^2))^{1/2}/P$$

The nonwoven fabric sheet 2 (elastic nonwoven fabric 1) elongated in the gear elongating step is sent to the cooling step in which the nonwoven fabric sheet 2 (elastic nonwoven fabric 1) elongated by means of the gear rolls 31 and 32 is cooled. In the cooling step, guide rollers 35 and 36 for guiding the elongated nonwoven fabric sheet 2 into a cooling equipment 37, the cooling equipment 37 for cooling the elongated nonwoven fabric sheet 2 while it is being conveyed in the form of a sheet, and guide rollers 38 and 39 for guiding the cooled nonwoven fabric 1 into the reeling step, in which the nonwoven fabric 1 is wound into a roll, are provided.

Since the elongated nonwoven fabric sheet 2 is rapidly cooled to approximately room temperature via means of the cooling equipment 37 in this step, plastic deformation of the stretchable fibers which contribute to the expression of elasticity of the elastic nonwoven fabric 1 after elongating, i.e. the polyurethane fibers, is effectively suppressed. As a result, the elastic nonwoven fabric 1 after gear elongating is effectively provided with an elasticity.

The elastic nonwoven fabric 1 cooled in the cooling step is reeled into a roll by means of a reeling equipment for reeling and is discharged as a elastic roll sheet.

Such an application of heat to the pre-elongation nonwoven fabric sheet 2 before changes the crystalline structure in the polyolefin fibers, thereby changing the orientation of the fibers. Accordingly, the degree of elongation of the fibers is increased, and the degree of elongation of the nonwoven fabric sheet 2 is likely to be increased. In addition, applying heat to the nonwoven fabric sheet 2 can prevent breaks in the nonwoven fabric sheet 2 in each elongating step.

Since the crystallinity degree of the olefin fibers is low and the distortion ratio of the olefin fibers is high, the range of elasticity is likely to be widened. Furthermore, since it is possible to obtain the nonwoven fabric sheet 2 with a high elongation ratio of fibers and sheets, a high elongation ratio can be applied during gear elongation and a wide range of elasticity can be obtained without weakening the sheet strength. This can prevent tearing of diapers, etc. when being pulled up during use even when disposable diapers are used, for example.

Since the use of elastic nonwoven fabrics with low basis weight is possible, in cases where disposable diapers are used, for example, the disposable diapers are easily stretched out when the diapers are expanded because the strengh of the diapers when the diaper s are stretched is reduced, and it is possible to reduce tightening of the gatherings during use, thereby producing diapers with high wearability.

Furthermore, since the fiber diameter of the olefin fibers is reduced and the fiber length is increased while the density of the elastic nonwoven fabric is decreased after gear elongation, elastic nonwoven fabrics with superior cushioning and breathability may be obtained.

Moreover, weakening in the strength of the nonwoven fabric sheet 2 may be reduced by applying heat treatment to the pre-elongation nonwoven fabric sheet 2 to increase the elasticity for elongation thereof. Accordingly, elongation processing may be conducted without fluffing, etc., even when elongation is being performed with a high elongation ratio at high speed, for example.

Moreover, in the present embodiment, heat is applied to the nonwoven fabric sheet 2 by machining the fabric through the heating rollers 33, in which heat generators are disposed as a means for applying heat to the nonwoven fabric sheet 2. However, heat may also be applied to the nonwoven fabric sheet 2 by applying high frequency energy, for example.

Moreover, the stretching direction of the elastic nonwoven fabric 1 can optionally be set according to the purpose of use of the elastic nonwoven fabric 1. More specifically, the nonwoven fabric sheet 2 can be elongated in either the width direction or longitudinal direction by arranging the nonwoven fabric sheet 2 in either the width direction or longitudinal direction relative to the direction in which the gear teeth 31t and 32t are arranged during gear elongation.

For example, when the pre-gear elongation nonwoven fabric sheet 2 is arranged in the width direction relative to the direction in which gear teeth 31t and 32t are arranged during gear elongation, an elastic nonwoven fabric 1 may be formed which can be stretched in the width direction. Similarly, when the pre-gear elongation nonwoven fabric sheet 2 is arranged in the longitudinal direction relative to the direction in which the gear teeth 31t and 32t,are arranged during gear elongating, an elastic nonwoven fabric 1 may be formed which can be stretched in the longitudinal direction. Furthermore, by performing gear elongation in the width direction and the longitudinal direction, it is possible to form a elastic nonwoven fabric 1 which can be elongated in both direction.

EXAMPLES

Hereinafter, the present invention will be explained in greater detail with reference to the Example(s). However, the present invention is not specifically limited to the below-mentioned Example(s).

Example 1

A nonwoven fabric sheet consisting of polypropylene fibers (PP) and polyurethane fibers (TPU) with a fiber diameter of 23 $\mu$m, a basis weight of 27 g/m$^2$, and a mixing ratio of 50% polyurethane fibers (TPU) was produced. The nonwoven fabric sheet was then subjected to gear elongating at a sheet temperature of the nonwoven fabric of 45°C and a machining speed of 50 m/min using gear rolls with a pitch P of 4.9 mm and an engagement length L of 4.0 mm, to obtain a elastic nonwoven fabric. The elastic nonwoven fabric had a basis weight of 30.38 g/m$^2$ and an elasticity range of 62.1%. The fiber diameter of polypropylene fibers (PP) in the low-density region 11 was 20 $\mu$m.
Change in Cushioning Property due to the Difference in Fiber Diameter and Basis Weight of the Elastic Nonwoven Fabric 1
A compressibility test was conducted by means of KES-FB3-AUTO-A, manufactured by Kato Tech Co., Ltd., with the obtained elastic nonwoven fabric as a test specimen. In the test, the measurement was conducted in a pressurized area of 2 cm$^2$, SENS 2, at compression speed of 50 sec/mm, and the compression workload WC during compression was calculated. Change in Breathability due to the Difference in Fiber Diameter and Basis Weight of the Elastic Nonwoven Fabric 1
Breathability resistance value was measured by means of KES-F8-AP1, manufactured by Kato Tech Co., Ltd., using the elastic nonwoven fabric obtained as a test specimen. The test specimen was mounted in a cylinder portion having a diameter of 28 mm, and the breathability resistance value was measured after 3 seconds of air discharge and after 3 seconds of air intake.

Table 1

| | | Example 1 | Example 2 | Example 3 | Example 4 | Example 5 | Example 6 | Example 7 | Example 8 | Example 9 |
|---|---|---|---|---|---|---|---|---|---|---|
| engagement length L | (mm) | 4.00 | 6.00 | 7.00 | 4.00 | 6.00 | 7.00 | 4.00 | 6.00 | 7.00 |
| fiber diameter of the polyolefin fibers | ($\mu$m) | 20 | 17 | 16 | 20 | 17 | 16 | 20 | 17 | 16 |
| basis weight | (g/m$^2$) | 30.38 | 31.43 | 28.15 | 38.48 | 38.51 | 35.67 | 52.13 | 55.98 | 50.34 |
| range of stretching | (%) | 62.10 | 125.50 | 164 | 64.50 | 120.90 | 155.7 | 49.50 | 119.50 | 149.5 |
| compression workload WC | (N*m/m$^2$) | 0.78 | 0.98 | 1.00 | 0.94 | 1.07 | 1.16 | 0.94 | 1.28 | 1.44 |
| breathability resistance value | (Kpa·sec/m) | 0.0104 | 0.0101 | 0.0118 | 0.0163 | 0.0130 | 0.0125 | 0.0331 | 0.0211 | 0.0265 |

Example 2

A nonwoven fabric sheet consisting of polypropylene fibers (PP) and polyurethane fibers (TPU) with a fiber diameter of 23μm, a basis weight of 27 g/m², and a mixing ratio of 50% polyurethane fibers (TPU) was produced. The nonwoven fabric sheet was then subjected to gear elongation at a nonwoven fabric sheet temperature 45°C, a machining speed of 50 m/min using gear rolls with a pitch P of 4.9 mm, and an engagement length L of 6.0 mm, to obtain an elastic nonwoven fabric. The elastic nonwoven fabric had a basis weight of 31.43 g/m² and an elasticity range of 125.5%. The fiber diameter of polypropylene fibers (PP) in the low-density region 11 was 17 μm. A test, similar to that of Example 1, was conducted by using the obtained elastic nonwoven fabric.

Example 3

A nonwoven fabric sheet consisting of polypropylene fibers (PP) and polyurethane fibers (TPU) with a fiber diameter of 23 μm, a basis weight of 27 g/m², and a mixing ratio of 50% polyurethane fibers (TPU), was produced. The nonwoven fabric sheet was then subjected to gear elongation at a nonwoven fabric sheet temperature of 45°C, a machining speed of 50 m/min using gear rolls with a pitch P of 4.9 mm, and an engagement length L of 7.0 mm, to obtain an elastic nonwoven fabric. The elastic nonwoven fabric had a basis weight of 28.15 g/m², and an elasticity range of 164%. The fiber diameter of polypropylene fibers (PP) in the low-density region 11 was 16 μm. A test as similar to Example 1 was conducted by using the obtained elastic nonwoven fabric.

Example 4

A nonwoven fabric sheet consisting of polypropylene fibers (PP) and polyurethane fibers (TPU) with a fiber diameter of 23 μm, having a basis weight of 35 g/m² and a mixing ratio of polyurethane fibers (TPU) of 50% was produced. The nonwoven fabric sheet was then subjected to gear elongating at a sheet temperature of the nonwoven fabric df 45°C and a machining speed of 50 m/min using gear rolls with a pitch P of 4.9 mm and an engagement length L of 4.0 mm to obtain an elastic nonwoven fabric. The elastic nonwoven fabric had a basis weight of 38.45 g/m² and an elasticity range of 64.5%. The fiber diameter of polypropylene fibers (PP) in the low-density region 11 was 20 μm. A test, similar to that of Example 1, was conducted by using the obtained elastic nonwoven fabric.

Example 5

A nonwoven fabric sheet consisting of polypropylene fibers (PP) and polyurethane fibers (TPU) with a fiber diameter of 23 μm, a basis weight of 35 g/m², and a mixing ratio of 50% polyurethane fibers (TPU), was produced. The nonwoven fabric sheet was then subjected to gear elongation at a nonwoven fabric sheet temperature of 45°C, a machining speed of 50 m/min using gear rolls with a pitch P of 4.9 mm, and an engagement length L of 6.0 mm, to obtain an elastic nonwoven fabric. The elastic nonwoven fabric had a basis weight of 38.51 g/m² and an elasticity range of 120.9%. The fiber diameter of polypropylene fibers (PP) in the low-density region 11 was 17 μm. A test, similar to that of Example 1, was conducted by using the obtained elastic nonwoven fabric.

Example 6

A nonwoven fabric sheet consisting of polypropylene fibers (PP) and polyurethane fibers (TPU) with a fiber diameter of 23 μm, a basis weight of 35 g/m², and a mixing ratio of 50% polyurethane fibers (TPU) was produced. The nonwoven fabric sheet was then subjected to gear elongation at a nonwoven fabric sheet temperature of 45°C, a machining speed of 50 m/min using gear rolls with a pitch P of 4.9 mm, and an engagement length L of 7.0 mm, to obtain an elastic nonwoven fabric. The elastic nonwoven fabric had a basis weight of 35.67 g/m² and an elasticity range of 155.7%. The fiber diameter of polypropylene fibers (PP) in the low-density region 11 was 16 μm. A test, similar to that of Example 1, was conducted by using the obtained elastic nonwoven fabric.

Example 7

A nonwoven fabric sheet consisting of polypropylene fibers (PP) and polyurethane fibers (TPU) with a fiber diameter of 23 μm, a basis weight of 50 g/m², and a mixing ratio of 40% polyurethane fibers (TPU), was produced. The nonwoven fabric sheet was then subjected to gear elongation at a nonwoven fabric sheet temperature of 45°C, a machining speed of 50 m/min using gear rolls with a pitch P of 4.9 mm, and an engagement length L of 4.0 mm, to obtain an elastic nonwoven fabric. The elastic nonwoven fabric had a basis weight of 52.13 g/m² and an elasticity range of 49.5%. The fiber diameter of polypropylene fibers (PP) in the low-density region 11 was 20 μm. A test, similar to that of Example 1, was conducted by using the obtained elastic nonwoven fabric.

Example 8

A nonwoven fabric sheet consisting of polypropylene fibers (PP) and polyurethane fibers (TPU) with a fiber diameter of 23 $\mu$m, a basis weight df 50 g/m$^2$, and a mixing ratio of 40% polyurethane fibers (TPU), was produced. The nonwoven fabric sheet was then subjected to gear elongation at a nonwoven fabric sheet temperature of the nonwoven fabric of 45°C, a machining speed of 50 m/min using gear rolls with a pitch P of 4.9 mm, and an engagement length L of 6.0 mm, to obtain an elastic nonwoven fabric. The elastic nonwoven fabric had a basis weight of 55.98 g/m$^2$ and an elasticity range of 119.5%. The fiber diameter of polypropylene fibers (PP) in the low-density region 11 was 17 $\mu$m. A test as similar to Example 1 was conducted by using the obtained elastic nonwoven fabric.

Example 9

A nonwoven fabric sheet consisting of polypropylene fibers (PP) and polyurethane fibers (TPU) with a fiber diameter of 23 $\mu$m, a basis weight of 50 g/m$^2$, and a mixing ratio of 40% polyurethane fibers (TPU), was produced. The nonwoven fabric sheet was then subjected to gear elongation at a nonwoven fabric sheet temperature of 45°C, a machining speed of 50 m/min using gear rolls with a pitch P of 4.9 mm, and an engagement length L of 7.0 mm to obtain an elastic nonwoven fabric. The elastic nonwoven fabric had a basis weight of 50.34 g/m$^2$ and an elasticity range of 149.5%. The fiber diameter of polypropylene fibers (PP) in the low-density region 11 was 16 $\mu$m. A test, similar to that of Example 1 was conducted by using the obtained elastic nonwoven fabric.

Evaluation

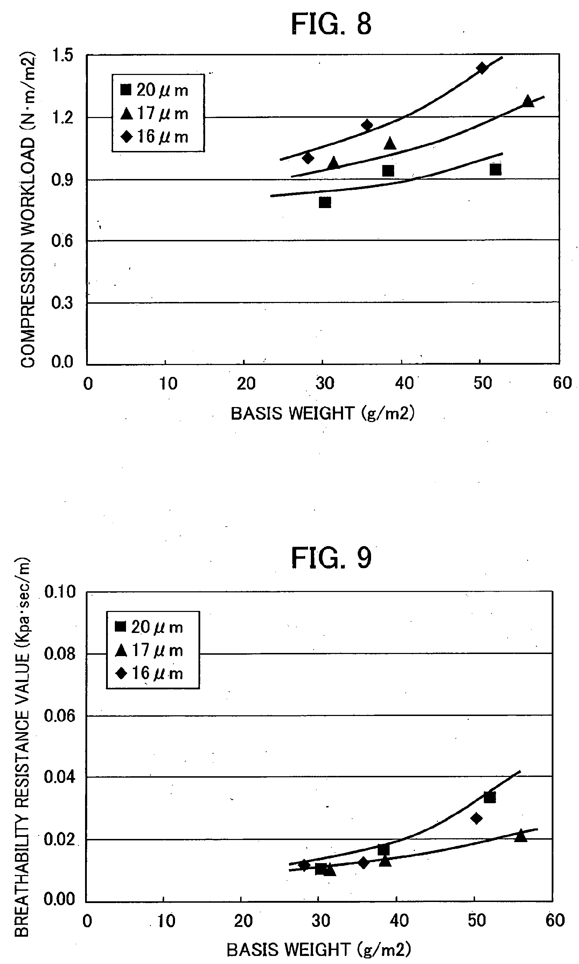

As shown in Fig. 8, a change in the fiber diameter of the olefin fibers causes the change in density of the elastic nonwoven fabric 1, and the void in the elastic nonwoven fabric 1 increases even though the basis weight remains the same. Based on the abovementioned results, the compression workload WC will also increase and the elastic nonwoven fabric is easily compressed, thereby increasing its cushioning property.

As shown in Fig. 9, a change in the fiber diameter of the olefin fibers causes the change in density of the elastic nonwoven fabric 1, and the breathability of the elastic nonwoven fabric 1 changes even though the basis weight remains the same. Accordingly, as the fiber diameter of the olefin fibers decrease, the void inside the elastic nonwoven fabric 1 increases, thereby reducing the value of breathability resistance. Therefore, the degree of breathability increases.

Example 10

Change of Strength under Conditions of Gear Elongating

A nonwoven fabric sheet consisting of 60% of polypropylene fibers and 40% of polyurethane fibers with a fiber diameter of 23 $\mu$m, and a basis weight of 35 g/m$^2$ was produced. The nonwoven fabric sheet 2 was then subjected to gear elongation in the flow direction F (machine direction) by using gear rolls 31 and 32 with a pitch P of 4.9 mm, and an engagement length L of 6.2 mm, to obtain an elastic nonwoven fabric 1. In the heating step, the sheet temperature was regulated to 48°C, by machining the nonwoven fabric sheet through the heating roller 33. The nonwoven fabric sheet had the maximum point strength of 45.9 N/50 mm in the machine direction.

The flow direction F of the equipment during production of nonwoven fabrics is the machine direction (MD).

An elastic nonwoven fabric 1 was produced at each speed, while changing the machining speed during machining. The obtained elastic nonwoven fabric 1 had a fiber diameter of polypropylene fibers (PP) in the low-density region 11 of 16 $\mu$m. The maximum point strength in the machine direction was tested using the obtained elastic nonwoven fabric 1.

Similarly, an elastic nonwoven fabric 1 was produced while changing the sheet temperature of the nonwoven fabric sheet. The obtained elastic nonwoven fabric 1 had a fiber diameter of polypropylene fibers (PP) in the low density region 11 of 16 $\mu$m. The maximum point strength in the machine direction was tested using the obtained elastic nonwoven fabric 1.

Evaluation

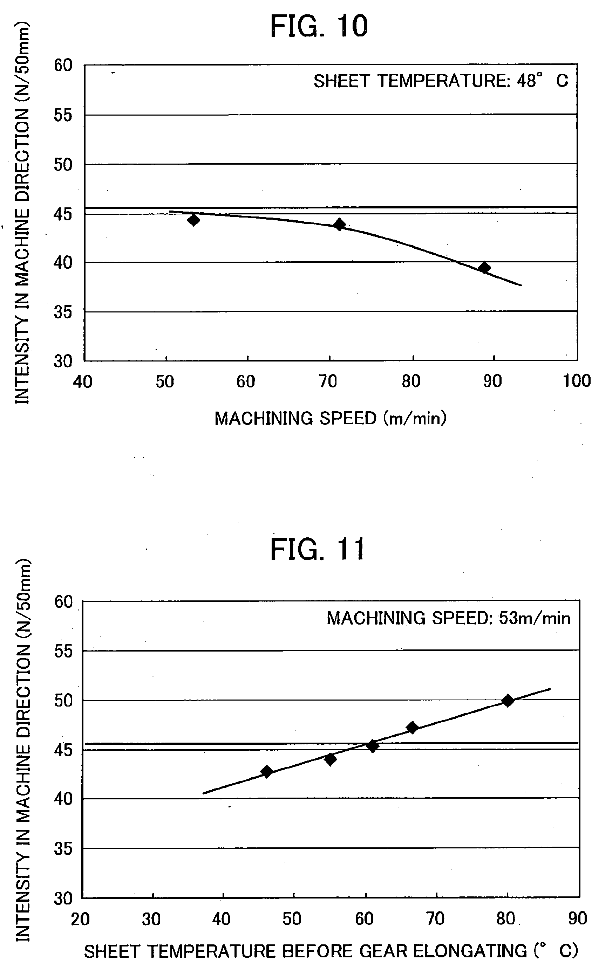

As shown in Fig. 10, as the machining speed is accelerated to 50 m/min., or more, the strength in the flow direction F is weakened to less than that of the nonwoven fabric sheet before gear elongating. However, as shown in Fig. 11, that the strength in the flow direction F can be increased even when the machining speed is 53 m/min., by applying heat to the nonwoven fabric sheet 2 during gear elongation.

**Claims**

1. An elastic nonwoven fabric (1) having a longitudinal direction and a width direction perpendicular to the longitudinal direction thereof, comprising:

   a plurality of belt-like low-density (11) regions extending in the longitudinal direction;
   a plurality of belt-like high-density regions (12) extending in the longitudinal direction, wherein the belt-like low-density regions (11) and the belt-like high-density regions (12) are formed on both faces of the elastic nonwoven fabric (1), alternately and continuously formed in the width direction, and wherein the belt-like high-density regions (12) on one face and the belt-like high density regions (12) on the other face are formed alternately in the width direction;
   thermoplastic polyolefin elongatable fibers (21); and
   thermoplastic elastomer stretchable fibers (22) mixed or layered with the thermoplastic polyolefin elongatable fibers (21),

   wherein the thermoplastic polyolefin elongatable fibers (21) include partially elongated fibers between the belt-like high-density regions (12) on one face and the belt-like high-density regions (12) on the other face, and the average fiber diameter of the fibers configuring the partially elongated fibers is 12 $\mu$m to 21 $\mu$m, and
   wherein a nonwoven fabric sheet (2), which is a nonwoven fabric with a latent elasticity, is subjected to elongation processing in order to form the elastic nonwoven fabric, the nonwoven fabric having latent elasticity contains thermoplastic polyolefin elongatable fibers and thermoplastic elastomer stretchable fibers (21, 22) mixed and fixed to one another by self-fusion induced by being softened or fused at plurality of fused portions (23) arranged at regular intervals.

2. The elastic nonwoven fabric (1) according to claim 1,
   wherein a basis weight is no more than 50 g/m$^2$.

3. A composite sheet (4) comprising:

   the elastic nonwoven fabric (1) according to claim 1 or 2;
   a non-elastic nonwoven fabric;
   a joint portion whereby the elastic nonwoven fabric (1) and the non-elastic nonwoven fabric are joined intermittently along a direction of stretching: and
   a non-joint portion between the joint portion and an adjacent joint portion in which a plurality of wrinkles are formed on a side not in contact with a skin along a direction intersecting the direction of stretching by relaxiation of a non-elastic sheet when the elastic nonwoven fabric (1) is not being elongated.

4. An absorbent article (50) comprising:

   a chassis (54) with front (55) and rear (56) portions, and an absorbent body (52) with liquid retaining capacity,
   wherein at least a part of the chassis (54) comprises the composite sheet (4) according to claim 3, and
   the composite sheet (4) thereof is arranged on a side in contact with the skin at a time the absorbent article is worn.

**Patentansprüche**

1. Elastischer Vliesstoff (1) mit einer Längsrichtung und einer Querrichtung, die senkrecht zur Längsrichtung ist, umfassend:

   eine Vielzahl von bandartigen geringdichten Bereichen (11), die sich in Längsrichtung erstecken,
   eine Vielzahl von bandartigen hochdichten Bereichen (12), die sich in Längsrichtung erstecken, wobei die bandartigen geringdichten Bereiche (11) und die bandartigen hochdichten Bereiche (12) auf beiden Seiten des elastischen Vliesstoffes (1), der abwechselnd und kontinuierlich in Querrichtung gebildet ist, gebildet sind, und wobei die bandartigen hochdichten Bereiche (12) auf der einen Seite und die bandartigen geringdichten Bereiche (11) auf der anderen Seite in Querrichtung abwechselnd gebildet sind;
   thermoplastische verlängerbare Polyolefin-Fasern (21), und
   thermoplastische elastomere dehnbare Fasern (22), die mit den thermoplastischen verlängerbaren Polyolefin-Fasern (21) gemischt oder aufeinandergelegt sind,

wobei die thermoplastischen verlängerbaren Polyolefin-Fasern (21) teilweise verlängerte Fasern zwischen den bandartigen hochdichten Bereiche (12) auf der einen Seite und den bandartigen geringdichten Bereiche (11) auf der anderen Seite umfassen, und der durchschnittliche Durchmesser der die verlängerten Fasern bestimmenden Fasern 12µm bis 21µm ist, und

wobei eine Vliesstofflage (2), die einer Vliesstofflage mit einer latenten Elastizität entspricht, einem Verlängerungsprozess unterzogen wird, um den elastischen Vliesstoff zu bilden, wobei der Vliesstoff mit der latenten Elastizität thermoplastische verlängerbare Polyolefin-Fasern und thermoplastische elastomere dehnbare Fasern (21, 22) beinhaltet, die vermischt und aneinander durch Selbstklebung, die durch Aufweichung oder Verschmelzung an einer Vielzahl von in geregelten Intervallen angeordneten Schmelzpunkten (23) erzeugt wird, befestigt sind.

2. Elastischer Vliesstoff (1) nach Anspruch 1, wobei ein Basisgewicht nicht größer ist als 50 g/m$^2$.

3. Compositelage (4) umfassend:

den elastischen Vliesstoff (1) nach Anspruch 1 oder 2;
einen nicht-elastischen Vliesstoff;
einen Verbindungsabschnitt, wobei der elastische Vliesstoff (1) und der nichtelastische Vliesstoff abwechselnd entlang einer Dehnrichtung verbunden sind;
und einen Nicht-Verbindungsabschnitt zwischen dem Verbindungsabschnitt und dem benachbarten Verbindungsabschnitt, in dem eine Vielzahl von Falten auf einer Seite, die nicht in Kontakt mit der Haut kommt, entlang einer Richtung, die die Dehnrichtung kreuzt, durch Entspannung einer nicht-elastischen Lage gebildet ist, wenn der elastische Vliesstoff (1) nicht verlängert wird.

4. Absorbierender Artikel (50) umfassend:

ein Chassis (54) mit vorderen (55) und hinteren (56) Abschnitten, und einen absorbierenden Körper (52) mit einer Flüssigkeitsaufnahmefähigkeit,
wobei zumindest ein Teil des Chassis (54) die Compositelage (4) gemäß Anspruch 3 umfasst, und
die Compositelage (4), wenn der absorbierende Artikel getragen wird, auf einer Hautkontaktseite angeordnet ist.

## Revendications

1. Tissu extensible non tissé (1) ayant une direction longitudinale et une direction transversale qui est perpendiculaire à la direction longitudinale, comprenant:

une pluralité de régions de faible densité en forme de bande (11) s'étendant en direction longitudinale,
une pluralité de régions à haute densité en forme de courroie (12), s'étendant dans la direction longitudinale,
lesdites régions de faible densité en forme de bande (11) et les régions à haute densité en forme de courroie (12) sont formées sur les deux côtés du tissu extensible non tissé (1) qui est en alternance et formées de manière continue dans la direction transversale, et dans lequel les régions à haute densité en forme de courroie (12) sont formées sur un côté et les régions de faible densité en forme de bande (11) sont formées sur l'autre côté en alternance dans la direction transversale sur l'autre côté;
des fibres extensibles polyoléfine thermoplastique (21), et
des fibres thermoplastiques élastomères expansibles (22), qui sont mélangés avec ou superposés les uns aux autres avec des fibres de polyoléfine thermoplastique extensible (21),

dans lequel les fibres thermoplastiques extensibles en polyoléfine (21) comprennent des fibres partiellement étendues entre les régions à haute densité en forme de courroie (12) d'un côté et les régions de faible densité en forme de bande (11) de l'autre côté, et le diamètre moyen des fibres qui déterminent des fibres étendues est de 12 microns à 21µm, et
dans lequel un couche de tissu non tissé (2), ce qui correspond à une un couche de tissu non tissé ayant une élasticité latente, est soumis un processus d'extension à former le tissu non-tissé élastique, dans lequel le tissu non tissé ayant l'élasticité latente comprend des fibres de polyoléfines extensibles thermoplastique et des fibres étirables élastomères thermoplastiques (21, 22) qui sont mélangées et fixés les unes aux autres par auto-adhésivité, qui est produite par le ramollissement ou la fusion d'une pluralité des points de fusion (23) prévues aux intervalles espacés dans le contrôle.

**2.** Tissu extensible non tissé élastique non tissé (1) selon la revendication 1, dans lequel un poids de base ne dépasse pas 50 g/m$^2$.

**3.** Couche composite (4) comprenant:

le tissu non tissée élastique (1) selon la revendication 1 ou 2;

un tissu non tissée non-élastique;

une partie de connexion, dans lequel le tissu non tissée élastique (1) et le tissu non tissée non-élastique sont reliées alternativement le long d'une direction de rotation; et une partie de non-liaison entre la partie de connexion et la partie de connexion adjacente, dans lequel une pluralité de plis est formée sur un côté qui ne vient pas en contact avec la peau, le long d'une direction croisant la direction d'allongement par dilatation d'une couche non-élastique lorsque le tissu non-tissé élastique (1) ne soit pas prolongé.

**4.** L'article absorbant (50), comprenant:

un châssis (54) ayant des parties première (55) et des parties arrière (56) et

un corps absorbant (52) ayant une capacité d'absorption des liquides,

dans lequel au moins une partie du châssis (54) comprend la couche composite (4) selon la revendication 3, et

la couche composite (4) est positionnée sur une page de contact avec la peau lorsque l'article absorbant est porté.

# FIG. 1

1

12      11  12      11
12

11  12

LONGITUDINAL
DIRECTION

WIDTH DIRECTION

# FIG. 2

21
23
22

## FIG. 3

RANGE OF STRETCHING (%)

ELONGATION RATIO OF NONWOVEN
FABRIC BEFORE ELONGATING (%)

## FIG. 4

STRENGTH

BEFORE GEAR ELONGATING

OLEFIN FIBERS

AFTER GEAR
ELONGATING

URETHANE FIBERS

DEGREE OF ELONGATION

ELONGATION RATIO

RANGE OF
STRETCHING

DISTORTION OF
URETHANE FIBERS

ELASTIC RECOVERY
OF OLEFIN FIBERS

# FIG. 5

FIG. 6

# FIG. 7A

WIDTH
DIRECTION

LONGITUDINAL
DIRECTION

# FIG. 7B

# FIG. 8

# FIG. 9

## FIG. 10

SHEET TEMPERATURE: 48° C

INTENSITY IN MACHINE DIRECTION (N/50mm)

MACHINING SPEED (m/min)

## FIG. 11

MACHINING SPEED: 53m/min

INTENSITY IN MACHINE DIRECTION (N/50mm)

SHEET TEMPERATURE BEFORE GEAR ELONGATING (° C)

## FIG. 12

# FIG. 13

LONGITUDINAL
DIRECTION

WIDTH DIRECTION

**EP 2 133 453 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004244791 A

- WO 2006115251 A1